# EUROPEAN PATENT SPECIFICATION

(11) **EP 4 259 223 B1**
(45) Date of publication and mention of the grant of the patent: **01.04.2026**
(21) Application number: 21908016.5
(22) Date of filing: 14.12.2021
(51) Int. Cl.: A61L 27/52, A61L 27/24, A61L 27/56, C08J 3/075, A61K 35/32, A61K 35/35, A61K 47/34, A61L 27/38

(54) **PARTICULATE MATERIALS FOR TISSUE MIMICS**
TEILCHENFÖRMIGE MATERIALIEN FÜR GEWEBE-MIMETIKA
MATÉRIAUX PARTICULAIRES POUR MIMÉTIQUES DE TISSU

(30) Priority: 14.12.2020 US 202063125280 P; 28.10.2021 US 202163263228 P; 24.11.2021 US 202163264564 P
(43) Date of publication of application: 18.10.2023
(73) Proprietor: THE REGENTS OF THE UNIVERSITY OF COLORADO, A BODY CORPORATE, Denver, CO 80203 (US)
(72) Inventor: BARTHOLD, Jeanne, Boulder, CO 80305 (US); NEU, Corey, P., Boulder, CO 80304 (US)
(74) Representative: Cabinet Chaillot
(86) International application number: PCT/US2021/072895
(87) International publication number: WO 2022/133429

(56) References cited:
- WO-A1-2019/140184
- WO-A1-2020/058456
- US-A1- 2020 268 944
- US-B2- 10 189 952
- US-B2- 10 463 768
- KIM YOUHWAN, KO HYOJIN, KWON IK KEUN, SHIN KWANWOO: "Extracellular Matrix Revisited: Roles in Tissue Engineering", INTERNATIONAL NEUROUROLOGY JOURNAL, vol. 20, no. Suppl 1, 26 May 2016 (2016-05-26), pages S23 - S29, XP055952999, DOI: 10.5213/inj.1632600.318
- MIDDLETON KELLIE K, VICTOR BARRO, BART MULLER, SATOSHA TERADA, FREDDIE H FU: "Evaluation of the Effects of Platelet-Rich Plasma (PRP) Therapy Involved in the Healing of Sports-Related Soft Tissue Injuries", THE IOWA ORTHOPAEDIC JOURNAL, vol. 32, 1 January 2012 (2012-01-01), pages 150 - 163, XP055953008
- LAUREN EDGAR, AFNAN ALTAMIMI, MARTA GARCÍA SÁNCHEZ, RICCARDO TAMBURRINIA, AMISH ASTHANA, CARLO GAZIA, GIUSEPPE ORLANDO: "Utility of extracellular matrix powders in tissue engineering", ORGANOGENESIS, vol. 14, no. 4, 2 October 2018 (2018-10-02), US , pages 172 - 186, XP055682964, ISSN: 1547-6278, DOI: 10.1080/15476278.2018.1503771
- BARTHOLD JEANNE E., ST. MARTIN BRITTANY M., SRIDHAR SHANKAR LALITHA, VERNEREY FRANCK, SCHNEIDER STEPHANIE ELLYSE, WACQUEZ ALEXIS, : "Percolation of Microparticle Matrix Promotes Cell Migration and Integration while Supporting Native Tissue Architecture", BIORXIV, 11 August 2020 (2020-08-11), pages 1 - 43, XP055953013, Retrieved from the Internet <URL:https://www.biorxiv.org/content/10.1101/2020.08.10.245589v1.full.pdf> DOI: 10.1101/2020.08.10.245589
- XIA HUIMIN; LI XIN; GAO WEIWEI; FU XIN; FANG RONNIE H.; ZHANG LIANGFANG; ZHANG KANG: "Tissue repair and regeneration with endogenous stem cells", NATURE REVIEWS MATERIALS, vol. 3, no. 7, 19 June 2018 (2018-06-19), London , pages 174 - 193, XP036553213, DOI: 10.1038/s41578-018-0027-6
- CONG YE, HAN XIAHE, WANG YOUPING, CHEN ZONGZHENG, LU YAO, LIU TINGJIAO, WU ZHENGZHI, JIN YU, LUO YONG, ZHANG XIULI: "Drug Toxicity Evaluation Based on Organ-on-a-chip Technology: A Review", MICROMACHINES, vol. 11, no. 4, pages 381, XP055854709, DOI: 10.3390/mi11040381

## Description

### CROSS-REFERENCE TO RELATED APPLICATIONS

This application claims the benefit of U.S. Provisional Application No. 63/125,280, filed December 14, 2020, U.S. Provisional Application No. 63/263,228, filed October 28, 2021, and U.S. Provisional Application No. 63/264,564, filed November 24, 2021.

### FIELD OF INVENTION

This invention relates to methods and devices for tissue engineering. More specifically, this invention relates to extracellular matrix tissue particle-based biomaterials and bioinks that can be used to create or 3D print layered tissues such as an articular cartilage scaffold.

### BACKGROUND OF THE INVENTION

The extracellular matrix of biological tissues is a dynamic environment that supports macroscale structure and protection for cells under physiological loading, while also providing microscale cellular contact for proliferation, differentiation, and receptor signaling. The specific structure and composition of both healthy and engineered extracellular matrix determines mechanical behavior and can guide cellular responses.

Biological tissues provide essential load bearing functions throughout the body. There are many remaining challenges in regenerative medicine, as some tissues like articular cartilage demonstrates an inability to repair following injury [Mow, V. C., Ateshian, G. A. & Spilker, R. L. Biomechanics of diarthrodial joints: a review of twenty years of progress. J Biomech Eng 115, 460-467 (1993)]. Tissues with high extracellular matrix content and low cell density require unique tissue engineering designs and approaches for robust and integrative repair solutions.

Healthy biological tissues are often composed of heterogenous and distinct tissue layers. For example, in the case of articular cartilage, each layer has a unique ratio of the two major extracellular membrane proteins; collagen and aggrecan. Each layer also has different cell densities. The composition of each cartilage layer and the interplay between layers allows articular cartilage to provide excellent joint lubrication and facilitates load transmission during normal movement.

Successfully replicating biological tissues with a tissue-engineered construct to repair defects following injury is an elusive challenge, despite many decades of innovation. To enable efficient and effective restoration of tissue defects, there exists a significant need to engineer a replacement scaffolds that mimic the structural properties of tissues and present natural biochemical cues for promoting cell growth and integration [Camarero-Espinosa, S., Rothen-Rutishauser, B., Weder, C. & Foster, E. J. Directed cell growth in multi-zonal scaffolds for cartilage tissue engineering. Biomaterials 74, 42-52 (2016)].

Patent application US 2020/268944 A1 discloses a composite biomaterial for tissue regenerative medicine comprising a gel resin in combination with decellularized tissue microparticles.

### SUMMARY OF THE INVENTION

This invention relates generally to a biomaterial, composed of (1) decellularized and particulated extracellular matrix and (2) thiolated polymers, as well as methods for the production and use of the biomaterial. Specifically, in one aspect the invention relates to treating areas of tissue disease or damage by combining the two biomaterial components and injecting the combined material into the damaged region. The invention can be used as a medical implant/treatment or as a biomaterial for 3D cell culture and/or 3D tissue printing. Methods for manufacturing the individual components, and the combined biomaterial are also provided herein.

Many tissues are layered with specific and complex heterogenous structures to provide critical functionality of the tissue. Many engineered constructs for *in vivo* tissue repair, 3D printing, organoid creation, lab on a chip, *in vitro* analyses, and lab grown meat fail to replicate the mechanics, unique layers, or complexity of a tissue and often rely on exogenous compounds to crosslink the materials into a stable scaffold. To address the need for mechanically robust, complex, and layered scaffolds, an extracellular matrix (ECM) tissue particle-based biomaterial ink has been developed which extrudes easily, polymerizes via disulfide bonding into a mechanically robust construct, and can be layered or used alone to create reliable tissue mimics for 3D organ culture or implantable medical devices for tissue repair. The ECM-based particle bioink, pECM bioink, utilizes functionalized macromolecules combined or packed together with acellular tissue particles. When combined, the two materials crosslink via disulfide bonding to create stable tissue mimics or repair scaffolds.

In particular, the invention relates to the composite material of functionalized macromolecules that can crosslink by disulfide bonding with acellular tissue particles. The functionalized macromolecules can be natural or synthetic. The tissue particles can be sourced from any animal or human tissue or organ. The sourced tissue particles can be mechanically broken down and decellularized prior to particle functionalization or in combination with the functional macromolecules. The invention also relates to the methods of combining the composite material and using the mixture to create a tissue mimic. There are numerous uses of the biomaterial disclosed herein, including applications for clinical tissue repair, such as an injectable/implantable medical implant (e.g., as repair for cartilage, skin, muscle, bone, kidney, liver, heart), tissue/lab on a chip for testing of pharmaceutical treatments (any tissue type), organoid development using the biomaterial mixed with living cells, as a commercial 3D printed biomaterial ink that can be used in series to create layered tissue mimics, and for labs/companies to answer a variety of questions (i.e., to serve as a model for tissues, disease, regeneration, etc.) needing an ECM based, or 3D culture, tissue system, and finally as a matrix to produce lab grown meat.

### BRIEF DESCRIPTION OF THE DRAWINGS

For a fuller understanding of the invention, reference should be made to the following detailed description, taken in connection with the accompanying drawings, in which:

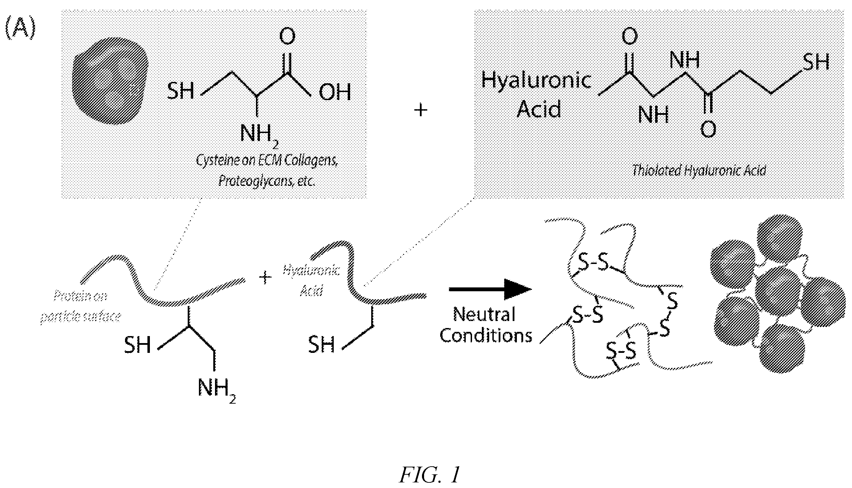

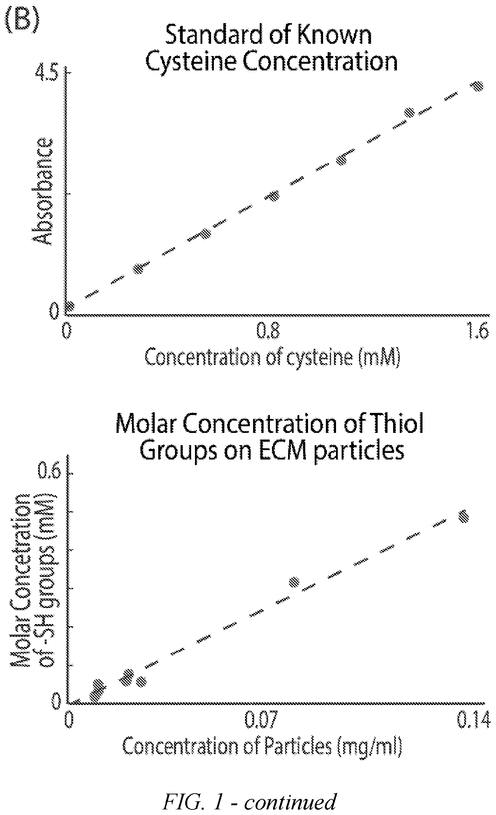

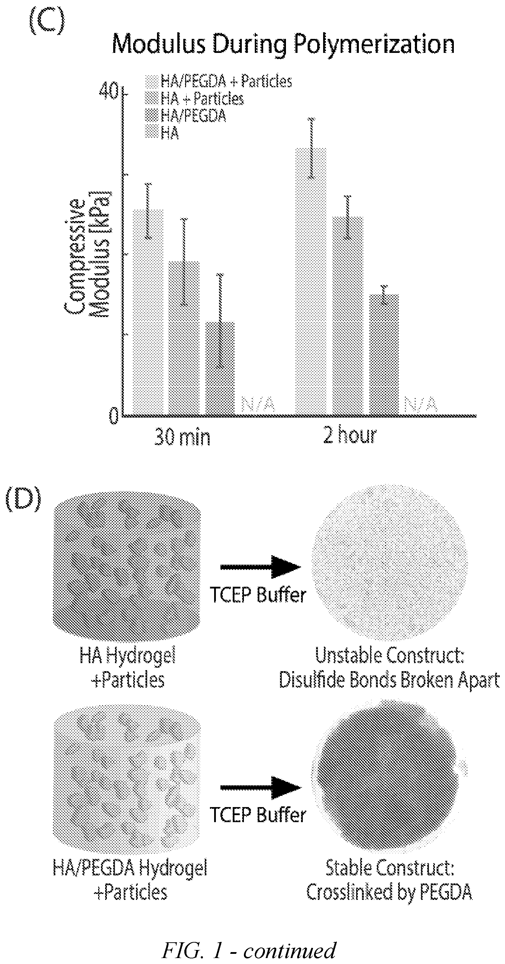

FIG 1 is a set of images and two graphs (Labeled (A)-(D)) illustrating the native tissue-based acellular biomaterial that crosslink ECM particles to functionalized macromolecules (here showing thiolated hyaluronan) via disulfide bonding. (A) Scaffolds crosslink by disulfide bond formation between thiolation sites on the macromolecule, in this example thiolated hyaluronan, and open sulfahydryl groups on the tissue particles thereby forming a stable 3D network. (B) By comparing to known quantities of cysteine, it was measured via Ellman's assay that particles contain sulfhydryl groups to interact with thiolated hyaluronan. (C) The addition of either particles, PEGDA, or both lead to initial cross linking and polymerization within 30 minutes, with further polymerization by 2 hours. The control containing only thiolated hyaluronan remained liquid at 2 hours, demonstrating the role the particles play in increasing the rate of crosslinking. (D) TCEP buffer is a reducing agent which breaks apart disulfide bonds. Incubation in TCEP buffer confirms that hyaluronan (HA) + particle gels crosslink via disulfide bonding.

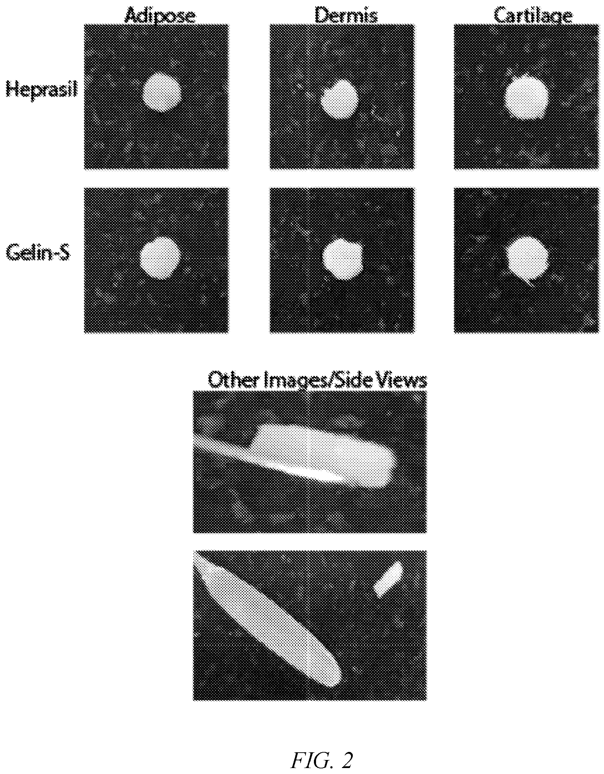

FIG. 2 is a set of eight images. Heprasil and Gelin-S are additional types of functionalized macromolecules (in addition to the thiolated hyaluronic acid/hyaluronan shown, for example, in FIG. 1). Heprasil is thiol-modified heparin/hyaluronic acid mix and Gelin-S is thiolated gelatin. When combined with the acellular microparticles (i.e., adipose, dermis, cartilage), all functionalized macromolecules cross-link with the acellular microparticles into stable and robust scaffolds.

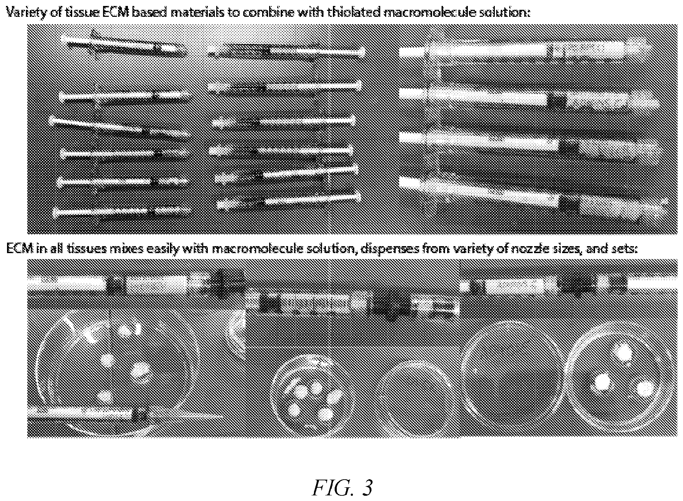

FIG. 3 is a set of images illustrating that the extracellular matrix can be sourced from a wide variety of tissue types, and then decellularized, and particulated for use as an ECM particle. Once in particle form, all tissues can be combined with functionalized macromolecules (in this example they are functionalized by thiol groups), mixed, and dispensed through any needle/nozzle size (limits depend on the largest size particle embedded in the ink).

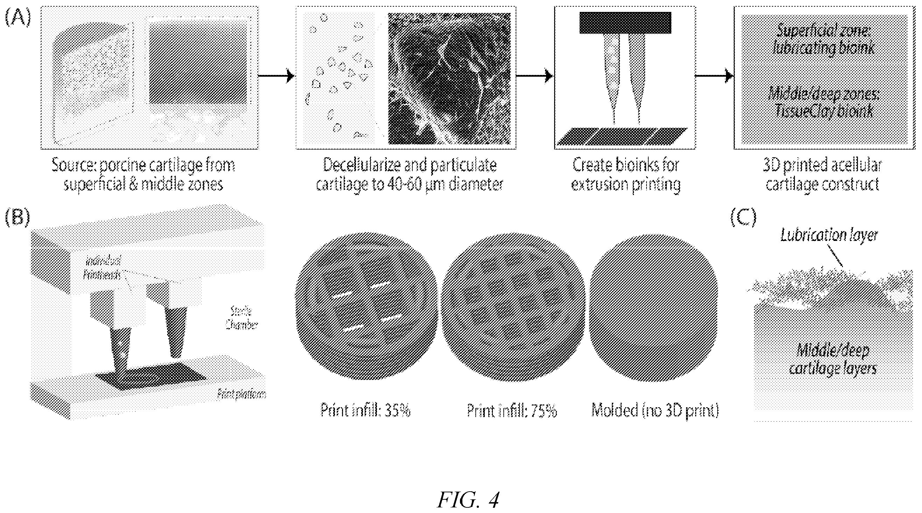

FIG. 4 is a set of illustrations depicting the use of ECM particle biomaterial as a bioink with layered printing for creation of cartilage mimics with mechanically-robust structure and lubrication. (A) In this illustrated process, cartilage tissue is decellularized and particulated to 40-100 µm. The resulting particles are encapsulated in hyaluronan and printed to create a mechanically robust cartilage scaffold. A top lubrication layer composed of only hyaluronan can be printed via a second nozzle to replicate lubrication surface characteristics of cartilage. (B) Scaffolds are printed at two different densities with a 22-gauge printhead tip to demonstrate the role of differentially sized macropores on nutrient diffusion, cell viability, and mechanics. For the purpose of comparison, a third group can be made by molding the scaffold, rather than printing. (C) The lubrication layer can be printed as a superficial layer to the cartilage tissue construct, where the superficial layer is composed of crosslinked hyaluronan, a polysaccharide which plays a key role in lubrication of native articular cartilage.

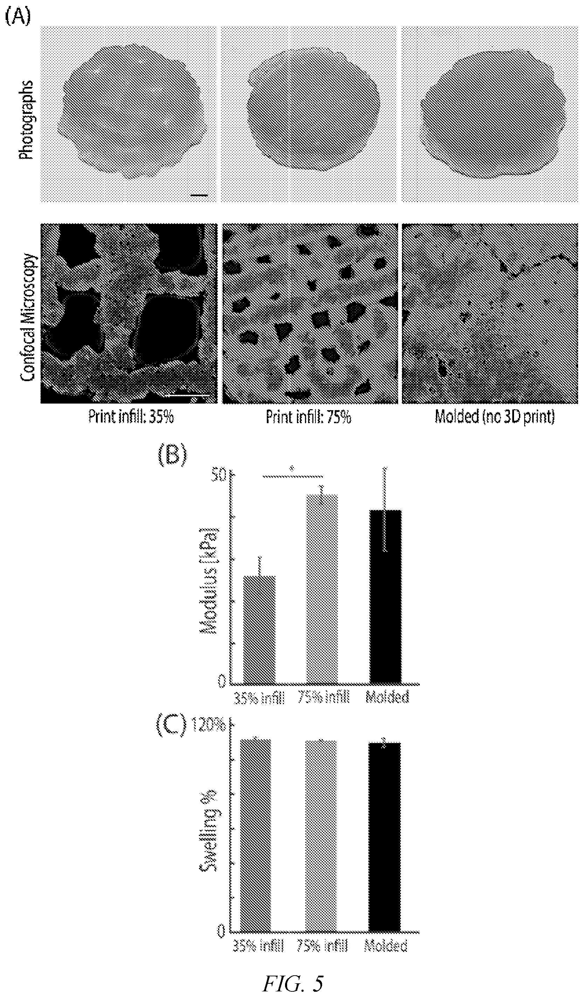

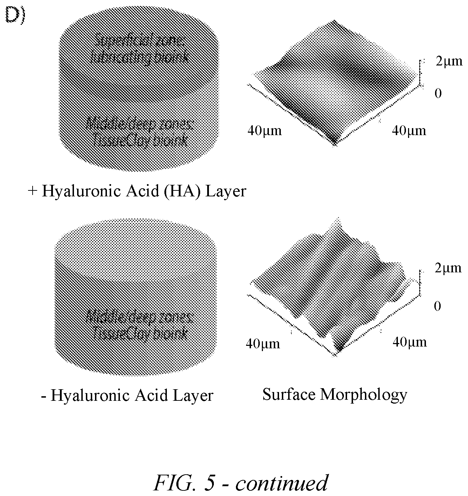

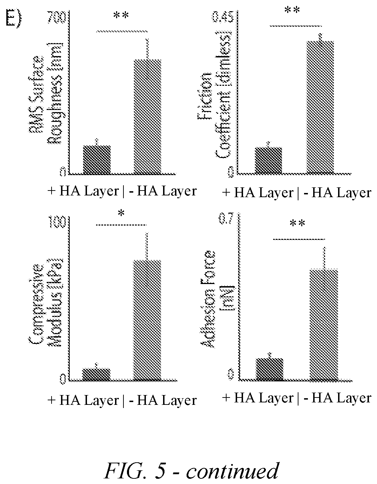

FIG. 5 is a set of illustrations, images and graphs demonstrating that, where biomaterial ink is 3D printed, the printing can be used to create layered tissue and specifically use unique acellular tissue inks to create tissue specific layering. When the biomaterial ink is printed via extrusion for articular cartilage, it can be used to create mechanically robust middle and deep zones with a smooth and low friction superficial zone. (A) Incorporating microtissue particles into a hyaluronan ink enables printing of a wide variety of structures. Here, scaffolds were created with a range of macropores: 1 mm, ~400 µm, no pores. (B) The addition of small macropores had no effect on the overall compressive modulus of the scaffold, measured under unconfined compression with a 10mm flat platen. (C) The incorporation of pores did not change the swelling levels of the constructs, confirming that the swelling depends heavily on the chemical crosslinking of the ink rather than the shape of the construct. (D and E) Printing a superficial zone of HA decreased the surface roughness, friction, and adhesion significantly (surface measured on the AFM with a 2µm spherical probe). * denotes p<0.05, ** denotes p<0.001, two-way ANOVA followed by Tukey's honest significant difference test. Scale bar in (A) is 1mm.

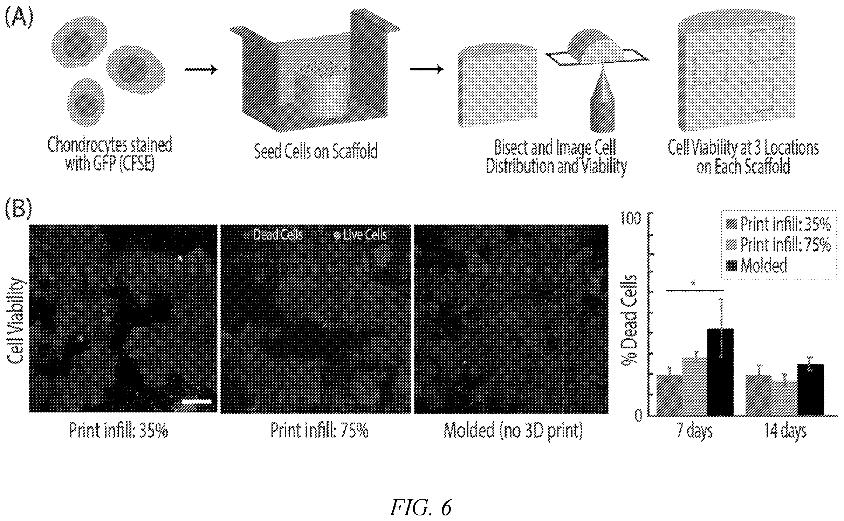

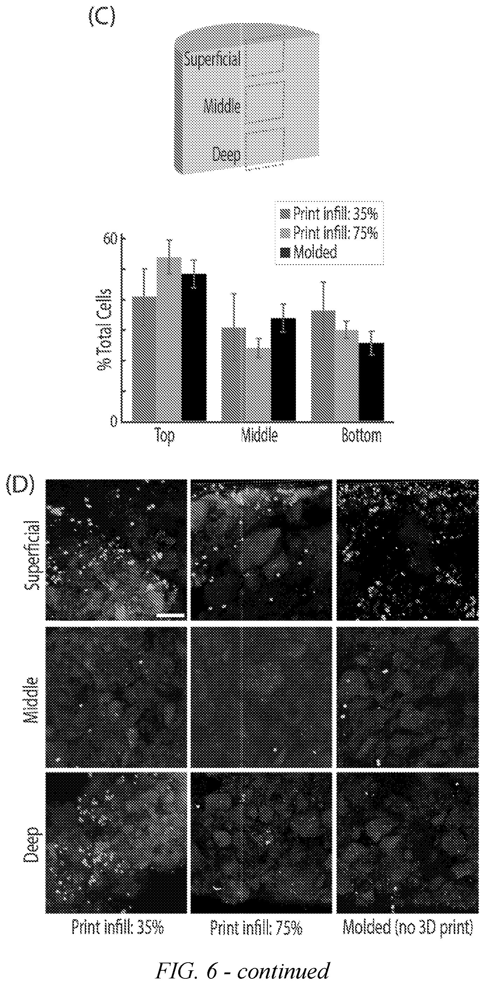

FIG. 6 is a set of illustrations, images and graphs demonstrating that, where biomaterial ink is 3D printed, the printing can be used to introduce a tissue scaffold with macropores. Increasing porosity in constructs led to improved cell distribution and viability. (A, B) Chondrocytes are extracted from bovine knee joints, stained with a fluorescent proliferation dye (CFSE), and seeded on top of all constructs. The percentage of dead cells was measured by quantification of CFSE (live) or ethidium homodimer (dead) stained cells present 7- and 14-days post seeding. (C, D) Chondrocyte distribution throughout the scaffold was measured by quantification of CFSE stained cells at three tissue depths: superficial, middle, and deep after 7 days of culture. *Denotes p<0.05, two-way ANOVA followed by Tukey's honest significant difference test.

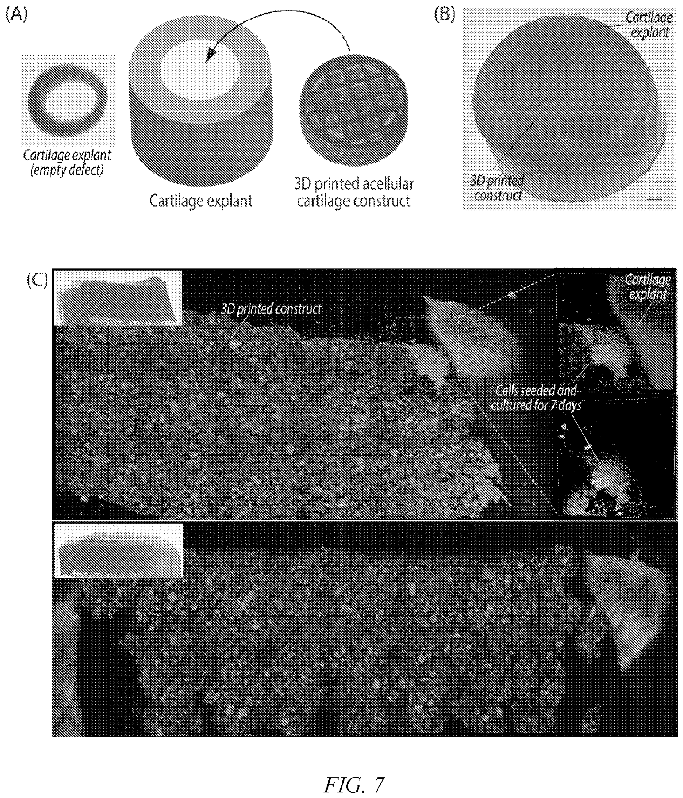

FIG. 7 is a set of illustrations and images showing that the biomaterial can be designed to be used in *in situ* to provide a 3D scaffold for studying tissue regeneration, repair, or barrier interactions/integration. The biomaterial can be designed and printed to fill a specific tissue defect and in the cartilage example shown, after 7 days the printed construct integrates and adheres to the surrounding native tissue. (A, B) Custom tissue defects are measured and inputted into CAD software to create a construct custom designed to fit the defect. Extrusion printing is used to create the custom construct and immediately introduced to the defect for polymerization. (C) After 7 days, constructs were bisected and imaged for direct observation of scaffold integrity and integration with native tissue. Scaffolds adhere to the native tissue, even with the forces exerted during bisecting. Additionally, CFSE stained cells (green - light gray in gray-scale), which were introduced after polymerization, collect where the native tissue and scaffold meet.

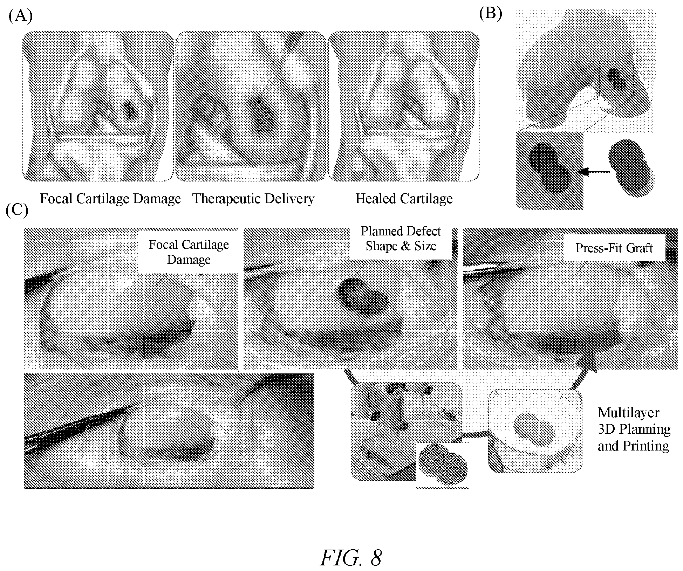

FIG. 8 is a set of illustrations and images showing that the 3D printing of ECM particle bioink is tunable for a wide variety of shapes and sizes and shows potential for clinical on-demand applications. (A) For clinical applications, it is essential to develop a material that can be applied to patient-specific defects, which can vary in terms of size and extent of focal cartilage damage within a complex joint. (B, C) With the ECM particle bioink, it is demonstrated that a custom defect shape can be measured, applied to a 3D CAD model, and printed via extrusion to create a multilayered construct suitable for implantation. Here, planning of a two-layer cartilage construct, using tissue particles for enhanced tissue structure in addition to an engineered lubrication layer, is printed and applied immediately to repair a joint defect.

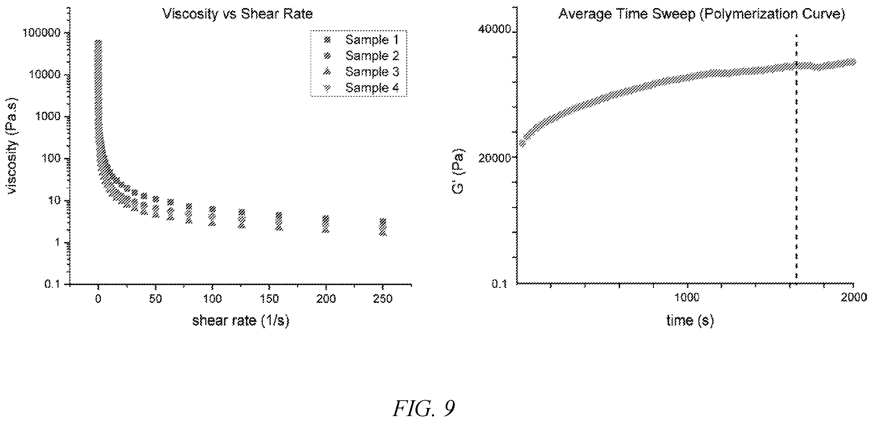

FIG. 9 is a pair of graphs showing that the ECM particle bioink can be evaluated for printability in numerous ways. Figure 9 shows the polymerization time (A) and shear thinning behavior (B) of the ECM particle bioink when using cartilage tissue, size 40-100 µm, and 20% thiolated hyaluronan. The rheological properties were measured using a Flow Procedure with a steady state flow step setting on Rheometer (AR-G2, TA Instruments). Experiments were carried out using parallel plates configured with 8-mm diameter plates in stiff bearing mode at room temperature with a gap distance of 800 µm. The shear sweep was measured as a function of shear rate from 0.01 to 200 s⁻¹ with no normal force, a pre-shear rate of 10 for 10s, and zero velocity. The time sweep was performed with parallel plates set at 37°C.

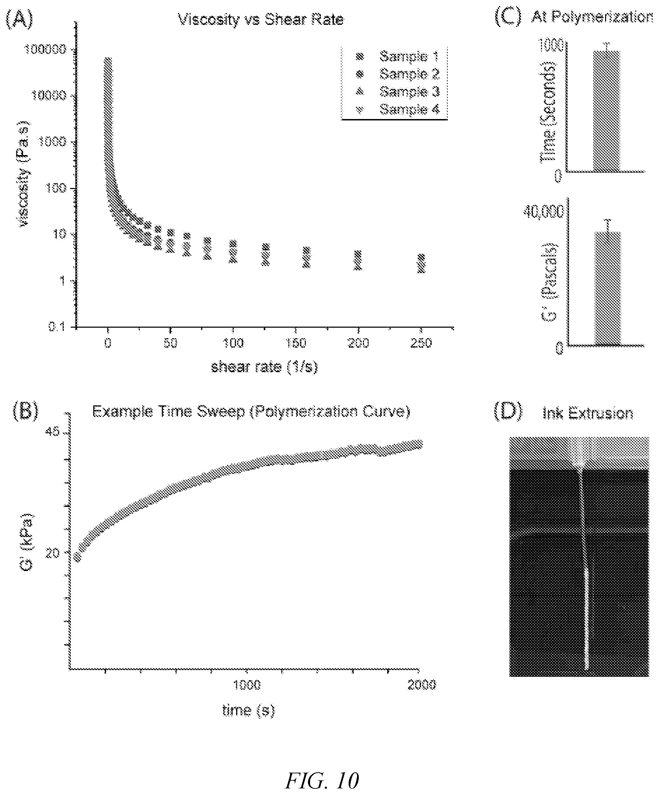

FIG. 10 shows the pair of graphs as shown in FIG. 9 (see FIGS. 10(A) and 10(B), along with two additional graphs (FIG. 10(C) showing the modulus at polymerization of the ECM particle bioink scaffold and the time it takes to reach a crosslinked state (for the graph to flatten) and FIG. 10(D) showing an image of the ink extrusion out of a 22 gauge nozzle used for 3D printing to demonstrate the smoothness and consistency of the extruded ink.

### DETAILED DESCRIPTION OF THE PREFERRED EMBODIMENT

A successful bioscaffold functions as a structural support for both cell integration and mechanical integrity. Ideally, an implantable bioscaffold is a substrate for cell attachment and proliferation, enables sufficient diffusion of nutrients and waste products through the scaffold, mimics native structural heterogeneities, and mechanically responds to normal loading on the specified tissue. Therefore, a decellularized tissue extracellular matrix (ECM) is a highly advantageous scaffold for tissue repair because it provides the same structural support as the native tissue, while additionally presenting familiar biological cues to enable cell invasion and integration from surrounding tissue *(in vivo)* or from introduced cells to the scaffold *(in vitro).*

However, in practice, the application of an intact whole decellularized tissue for tissue defect repair is limited by shape constraints and can be limited by high native tissue density (depending on the tissue needing to be replicated). The ECM from decellularized donor tissue can be broken down into small particles, and then fabricated into a composite material by densely packing particles in a hyaluronic acid/PEGDA crosslinked hydrogel to create a regenerative model for cartilage repair. The dense packing of particles closely recapitulates mechanical properties of large decellularized tissue, but also significantly improves cellular infiltration into the scaffold. While hydrogels packed with ECM particles are a promising advancement in scaffold technology, it is critical to produce a scaffold that also mimics the distinct layered structure of tissues (e.g., articular cartilage), and uses a crosslinking chemistry that does not depend on the Michael addition reaction between functionalized hyaluronic acid and PEGDA diacrylate groups.

The present invention addresses problems with prior bioscaffolds by creating a scaffold that uses a crosslinking interaction between functionalized macromolecules (natural or synthetic) and tissue particles directly.

Advanced manufacturing techniques, especially 3D printing, provide several advantages for tissue engineering and bioscaffold fabrication. A wide variety of applications utilize extrusion-based printing, enabling the tunability of the extrusion size, use of a wide range of printable materials, the capacity to create constructs with unique layers, and the ability to print custom shapes and sizes. Prior attempts have involved extrusion-based printing of conventional synthetic polymers to create tissue engineered scaffolds. as these polymers provide enhanced engineering control. Unfortunately, synthetic polymers alone generally fail to fully mimic the structure or function of most natural tissues. Alternatively, natural polymers, often composed of proteins occurring in abundance in the body such as hyaluronan, alginate, and collagen, have been used for engineered 3D printed tissue applications. Unfortunately, most natural polymer-based hydrogels rely on UV light and other extra photoinitiators to crosslink and form a stable scaffold, which additionally do not recapitulate the complex interplay between the proteins and polysaccharides found in most native tissue. To increase the polymer complexity of natural hydrogels, researchers have developed extracellular matrix-based bioinks by digesting decellularized tissue into liquid (monomeric) form. [J. S. Choi, H. J. Yang, B. S. Kim, J. D. Kim, J. Y. Kim, B. Yoo, K. Park, H. Y. Lee, Y. W. Cho, Journal of Controlled Release 2009, 139, 2.] In these procedures, proteases digest the tissue and break down the rigid ECM structure, which liquifies the tissue. Unfortunately, the tissue digestion also destroys the protein organization that provides the tissue with its unique structure, ECM architecture, protein interactions, and cellular attachment sites; essential components to creating an ideal regenerative tissue environment.

Here, a material composition of an acellular tissue-based biomaterial has been developed that can be injected into a defect or damaged region in a patient (human or animal) or be used as a 3D printing biomaterial resin (pECM bioink) to create architecturally robust tissue mimics for 3D organ culture or tissue-on-a-chip applications. In one embodiment, the biomaterial is sourced from purely tissue-based and natural extracellular matrix components. In other embodiments, the natural tissue matrix is crosslinked with functionalized synthetic polymers. The method and material composition taught herein leverages the sulfhydryl groups on cysteines of decellularized tissue to crosslink with functionalized macromolecues, creating disulfide bonds between the two biomaterial ink components. Unlike the biomaterial inks formed from complete tissue digestion, acellular microparticles are produced via mechanical pulverization, resulting in ECM microparticles that preserve the micro mechanical and biological properties of the original tissue. The pulverized particles can be densely packed in a hydrogel solution of functionalized macromolecules and injected/applied to any shape defect or printed via extrusion. Using techniques employed such as in viscous clay printing, a printed ECM particle biomaterial ink was developed. We demonstrate that printing with ECM particle biomaterial ink enabled the fabrication of layered tissues and the ability to print macro pores in the tissue mimics. Additionally, we illustrate the simplicity in printing and implanting custom scaffolds into a patient using a layered, and lubricated, articular cartilage scaffold printed to a custom shape and implanted into a defect.

The present invention provides tissue extracellular matrix particles obtained from a tissue or organ from an animal or human. The extracellular matrix particles are mechanically pulverized to a selected size. In various embodiments, the particles can be 0-100 µm, 100-250 µm, or 250-500 µm. The particles are decellularized using chemical means to remove the cellular components of the tissue, lyophilized, and size sorted into the different size categories. The extracellular matrix particles after decellularization include native growth factors, attachments sites for cells, which will promote cellular interaction, and regeneration cascades, and complex tissue-specific architecture.

The particles are combined with a non-ECM particle component: a polymer (either natural or synthetic, preferably natural) which is functionalized by thiolation (i.e., to have open thiol (-SH) groups), and dissolved for easy mixing with the particles. In different embodiments, the percentage of the molecular groups on the macromolecule which are functionalized, or broken apart to have open thiol groups, can fall into one of the following categories: 0-15%, 15-30%, and/or 30-60%. The functionalization percentage dictates the crosslinking density of the combination material, where a higher level of cross-linking results in smaller pores/lower material porosity and stiffer materials.

### Example 1 - Materials and Methods - Particulate Tissue Extracellular Matrix Crosslinked with Thiolated Polymers Creates Scaffold

### Sourcing Tissue ECM:

Tissue ECM is most often obtained from an animal or a human after a terminal procedure, but can also be obtained during a procedure involving tissue replacement or tissue extraction (e. liposuction). Tissue is harvested either freshly after harvest (within 48 hours of sacrifice) or is sourced as frozen tissue (which was flash frozen within 48 hours of sacrifice or harvest). Tissue is collected from the tissue of interest using a scalpel and/or dissection scissors. It is critical to not harvest the tissue in a region where it meets a different type of tissue (at the edges) to ensure that only the desired tissue type is harvested. After harvest, tissue is cut into ~1 cm² pieces, prior to flash freezing and storing or tissue grinding. Tissue can be sourced from xenogenic, allogenic, or autogeneic sources and can be stored as tissue from a single source (e.g., for personalized medicine or an autograft), or can be pooled with other samples of that tissue type for more generalized use.

### Tissue Grinding:

Tissue is ground at two stages of the fabrication process: Stage 1. After extraction from the tissue or organ (while it is still composed of cellular contents) and Stage 2. After decellularization and lyophilization. At stage 1, flash frozen tissue pieces are enclosed in flash frozen stainless steel grinding jars with a free floating stainless steel sphere. The grinding jars with tissue are pulverized at a high rate of vibration (e.g., 30 cycles/second on a TissueLyser II grinder) for 30 seconds-1 min. At stage 2, after decellularization, lyophilized acellular cartilage tissue particles are pulverized further in stainless steel grinding jars (room temperature grinding jars and tissue) at the same rate (e.g., 30 cycles/second on a TissueLyser II grinder) for 60 seconds. Following the second pulverization, particles are size sorted using a vibrational sieve (e.g., one supplied by Endecott) into distinct sizes: <40 µm, 40-100 µm, 100-250 µm, and 250-500 µm.

### Decellularization:

Microparticles are decellularized using a sequence of chemical and enzymatic means. After the first bulk tissue grinding, cells are broken down and stripped away from the tissue by first submerging in 2% Sodium Dodecyl Sulfate (SDS) for 8 hours under agitation at 37°C. Particles are then rinsed overnight in 1X Phosphate Buffered Saline to remove the SDS. Optionally, tissue particles can then be submerged in an enzymatic treatment (e.g., 0.1% DNase for 3 hours) under slow agitation at 37°C to further breakdown leftover cellular and genetic material. Microparticles are rinsed in PBS, flash frozen in liquid nitrogen, lyophilized, and stored frozen at -80°C until the second round of grinding (detailed above). The extracellular matrix is stored substantially free of cells and can be described as a collection of small particles. These particles, in combination with functional macromolecules, may be used for research purposes or sterilized, stored, and processed into an implant.

### Functionalization of Polymers:

Numerous polymers can be functionalized to have open -SH groups that can perform disulfide bonding with -SH groups on tissue ECM. One example of macromolecule functionalization is the thiolation of a hyaluronic acid molecule. For this example, glucoronate carboxyl groups on hyaluronan (HA) were replaced with thiol groups following previously established protocols [D. Eng, M. Caplan, M. Preul, A. Panitch, Acta Biomaterialia 2010, 6, 2407]. Briefly, hyaluronan (MW 100 kDa, Lifecore Biomedical) was dissolved at 10 mg/ml in degassed milliQ water. Dithiobis propanoic dihydrazide (DTP) (Frontier Scientific) was added to the solution, the pH was lowered between 4.5 and 4.75, and then (1-ethyl-3-(3-dimethylaminopropyl) carbodiimide (EDC) (ThermoFisher) was added to begin the reaction. The pH was maintained between 4.5 and 5 for 50 minutes. After 50 minutes, the reaction was stopped by raising the pH above 7. Next, dithiotreitol (DTT) (Fisher Scientific) was added, the pH was raised above 8.5, and the solution was stirred for 24 hours at room temperature. After 24 hours, the pH of the solution was lowered, the liquid transferred to dialysis tubing (10 kDa membrane cutoff, Spectrum Labs) and the solution was dialyzed in an HCl solution supplemented with 100 mM sodium chloride for 8 solution changes, then in an HCl solution without supplements for 4 solution changes. Dialysis was completed in a sealed chamber with continuous nitrogen gas bubbling into the HCl solution. With each batch of HA thiolation, the substitution rate can be confirmed to be in the defined invention ranges of 0-15%, 15-30%, or 30-60% (thiolated mmols/unthiolated mmols) using a standard Ellman's assay following manufacturer's protocol (Ellman's solution, ThermoFisher).

### Combine with Functionalized Polymer:

Size sorted, and acellular lyophilized tissue particles are combined with a functionalized polymer (either purchased or prepared as described above, and either natural or synthetic) to create the composition biomaterial. Lyophilized and functionalized polymers (see above), dissolve easily when introduced to 1X PBS, and are dissolved to create an aqueous solution of twice the desired polymer concentration (for example, 20 mg/ml of macromolecule) which is neutralized to a pH of 7 and loaded into a syringe, taking care to remove any bubbles that formed in syringe loading. In a separate syringe, acellular size sorted tissue particles are mixed with 1X PBS at twice the desired final ratio (for example, 0.4 g/ml). Once ready to combine (e.g., for device delivery in a defect, for tissue bioprinting, or for 3D cell culture), syringes are attached to each other using a custom luer lock connector with cross hairs across the opening to facilitate mixing. Solutions are syringed back and forth approximately 30 times until mixing is complete and the material runs smoothly through the luer lock mixing connector. The final mixture should have a macromolecule concentration roughly half of what each individual component had (in the example throughout, it would be 10 mg/ml and particle concentration of 0.2 g/ml).

### Example 2 - Therapeutic and R&D Use

The invention provides acellular tissue particles (either sourced from one tissue or multiple tissues and combined) crosslinked to thiolated polymers to repair and provide a regenerative scaffold for defective, damaged, diseased, or missing tissues or components of an organ in a subject in need (either animal or human subject). In certain embodiments, the material composition may be used to treat defective or damaged organs which include, but are not limited to, articular cartilage, muscle, skin, ligaments, subcutaneous adipose beneath skin, kidney, and liver.

In particular, the biomaterial composition may be used to treat diseases, conditions, or damage that are associated with bulk tissue loss and that may benefit by a structurally mature regenerative tissue scaffold to immediately fill a volume, provide mechanical function of the damaged tissue, and promote cells to migrate into the material, attach, and proliferate in a regenerative cascade. Such diseases would include, but are not limited to facial wasting syndrome, articular cartilage damage due to injury, osteoarthritis, ulcers, large or deep burns and/or wounds, breast reconstructions, volumetric muscle loss, and impaired vocal folds.
In certain embodiments, the material composition may be used as a biomaterial ink extruded using 3D printing techniques. Applications for using this material for 3D printing would include, but are not limited to, tissue-on-a-chip, tissue mimics, 3D cell culture, personalized medicine, personalized treatment testing, drug toxicity testing, and barrier tissue crosstalk research.

### Example 3 - Medical Devices

The biomaterial composition can be formulated as a medical device for injection and/or permanent implantation into a subject. The medical device would use the solubilized polymer as a carrier for the extracellular matrix tissue particles to supply the material to a region of need. More specifically, the biomaterial composition will be applied to a region of defect or damage to promote regeneration and/or provide structural stability in the damaged region.

In some embodiments, the polymer can also be mixed with external growth factors, region specific anti-inflammatory drugs, or other necessary active and FDA-approved pharmaceutical agents (e.g., TGF β, IGF-1, PRP, corticosteroids, etc.), prior to being mixed with the extracellular matrix particles and injection to the desired site. The ingredients of the compositions of the invention can be supplied packaged separately and mixed prior to administration.

Thiolated polymers used to compose the biomaterial composition should be biocompatible and avoid irritation or systemic reactivity to native tissue/host. Furthermore, it is possible to combine multiple functionalized polymers (for example, hyaluronan and collagen) with the extracellular matrix particles, if desired. In preferred embodiments, a single type of functional polymer is used.

### Example 4 - Tissue Regeneration Scaffold

One important aspect and application of the invention is the use of extracellular matrix particles combined and crosslinked with thiolated polymers to create an ideal scaffold for tissue regeneration. In an advantageous embodiment, the scaffold is injected into an area of the body where it crosslinks and adheres to surrounding native tissue via activated functional groups on the macromolecule. The growth factor remnants in the extracellular matrix particles encourage cell migration and attachment. As the cells in the tissue and the surrounding tissue begin to multiply and/or lay down new extracellular matrix, the gaps between the tissue particles are filled in with newly grown tissue. In another embodiment, the biomaterial composition described herein can be formed in sterile conditions *in vitro.* Cells can be seeded in the scaffold slurry before or after crosslinking. The cellular scaffold can be maintained and cultured until it reaches a desired tissue maturity, at which point the regenerative scaffold can be implanted into the patient (e.g., as an advancement to procedures such as matrix-assisted autologous chondrocyte implantation, where tissues or cells must be cultured outside of the patient.)

### Example 5 - Cell Culture Substrate

Many cells require or exhibit enhanced growth on substrates other than tissue culture plastic to grow and retain proper function outside their native tissue environments. In particular, cells which rely on their surroundings and external signaling for differentiation require non-plastic substrates (e.g., stem and pluripotent cells). In certain embodiments, the biomaterial composition taught herein can be used for cell culture of a variety of cells. The tissue source of the particles will dictate the specific extracellular matrix environment of the cell culture, potentially providing signals to differentiate stem cells or maintain cells in a tissue specific phenotype. In certain embodiments, cells can be plated on the surface of the crosslinked biomaterial invention described herein. In other embodiments, the cells can be encapsulated in the thiolated polymer, mixed with the particles as described, and plated in a mold for a 3D culture environment.

### Example 6 - Printing for personalized medicine or 3D cell culture

CAD designs for the specific defect shape or desired 3D culture model should be loaded in the 3D printer, and the use should adjust infill density accordingly to the application. The freshly mixed biomaterial ink can be loaded into printing cartridges and the print should be started within 5-10 minutes of mixing. The ink should be kept at 4°C to prevent preliminary crosslinking, and the print should take no more than 30 minutes.

### Example 7 - Scaffold for Lab Grown Meat Alternatives

In certain embodiments, the material composition may be used as a tissue specific substrate to grow meat or meat substitutes artificially in the lab. The material can be used to differentiate cells into tissue specific cells (e.g., muscle) and, as such, the scaffold promotes new tissue matrix production and potential to produce meat alternatives.

### Example 8 (Part 1) - Cartilage scaffold with hyaluronic acid crosslinked to hyaline cartilage particles

Cartilage microparticles, which were decellularized, morsilized, and lyophilized, were encapsulated in thiolated hyaluronic acid (HA) hydrogels at a ratio of 0.2 g/ml, via mixing of two groups: 1. Lyophilized particles in PBS and 2. Functionalized HA, dissolved in PBS and neutralized to pH 7. HA had previously been thiolated to 22%, and dissolved easily when introduced to sterile DPBS. The hydrogels were incubated at 37°C for 30 minutes to facilitate disulfide bond crosslinking of the open sulfide groups on the cysteine of particles with the thiol groups on the functional HA molecules to form a stable 3D structure. It is contemplated that incubation temperatures can be varied around a roughly optimal temperature of 37°C (e.g., 32°C to 42°C). For example, decreasing temperatures, such as to room temperature, will require increased incubation times.

### Example 8 (Part 2) - Kidney proximal tubule tissue mimic

In another example, to print a kidney proximal tubule model, kidney from a porcine source was used to make freshly prepared kidney pECM bioink (e.g., including thiolated HA), particle sizes <100 µm. The ink was printed using direct ink writing on a sterile multi-nozzle extrusion printer (BioX, Cell Ink Life Sciences), in parallel with a sacrificial ink which was used to print the tubule channels. ECM particle bioink was extruded using a pneumatic print cartridge and a 22-gauge nozzle into a custom PDMS mold. Ink was extruded with 40-60 kPa pressure (adjusted in real time to ensure print consistency between layers) at a rate of 5 mm/s. The ink was set to pre-flow and post-flow for 30ms before and after print steps. Nozzles were calibrated prior to printing to ensure alignment of the multiple printheads and unique inks. After printing and cross-linking, scaffolds are cooled to liquify the sacrificial inks, and perfusion is used to remove the liquid.

### Example 8 (Part 3) - 3D Print-layered bone-cartilage in vitro

In a further example, to print an osteochondral (bone and articular cartilage) scaffold to fill a custom knee defect, the following procedure was followed: pECM bioink (e.g., decellularized cartilage particles in PBS solution mixed with thiolated HA solution) using either bone or cartilage extracellular matrix, freshly prepared, was printed using direct ink writing on a sterile multi-nozzle extrusion printer (BioX, Cell Ink Life Sciences). 3D print designs were created in open-source CAD software (OnShape), following the measurements of the defect in the injured knee and transferred to the 3D printer for segmenting. ECM particle bioink was extruded using a pneumatic print cartridge and a 22-gauge nozzle. Ink was extruded with 70-90 kPa pressure (adjusted in real time to ensure print consistency between layers) at a rate of 2 mm/s. The ink was set to pre-flow and post-flow for 30ms before and after print steps. Ink was printed with a 75% infill using a grid pattern. For the top lubrication layer, the superficial zone ink was printed with 3-10 kPa of pressure and a speed of 20 mm/s with no pre or post flow. Nozzles were calibrated prior to printing to ensure alignment of the multiple printheads and unique inks. Immediately post-printing, the construct was inserted into the defect region to promote polymerization post-implantation, a key process for native tissue integration. See, for example, FIG. 8C.

### Example 8 (Part 4) -3D print layered scaffold in situ

In another example, the same procedure as example 8, part 3 is followed, with printing directly into the knee defect, and polymerized (37 °C, 30 minutes; 18 minutes to crosslink based on rheology, but incubated 30 minutes to allow for minor differences in environment) in the joint using all sterile components, preparation, and surgical suite.

### Example 8 (Part 5) -Dermis scaffold with gelatin crosslinked to dermis particles

In another example, porcine dermis tissue was ground into small pieces, which were decellularized, morsilized, and lyophilized. The decellularized particles were combined with a Gelin-S (sourced from Advanced Biomatrix), which is a form of thiolated gelatin, at a ratio of 0.2 g/ml, via mixing of two groups: 1. Lyophilized particles in PBS and 2. Gelin-S, dissolved in PBS and neutralized to pH 7. The hydrogels were incubated at 37°C for 30 minutes to facilitate disulfide bond crosslinking of the open sulfide groups on the cysteine of particles with the thiol groups on the gelatin molecules to form a stable 3D structure composed of dermis ECM and collagen I, with extracellular matrix components that would provide for healthy skin following wound repair.

### Example 8 (Part 6) - Bone or cartilage scaffold with heparin sulfate

In another example, porcine bone and hyaline cartilage tissue was ground into small pieces, which were decellularized, morsilized, and lyophilized. The decellularized particles were combined with Heprasil (sourced from Advanced Biomatrix), which is a combination of thiolated heparin sulfate and thiolated hyaluronon, at a ratio of 0.2 g/ml, via mixing of two groups: 1. Lyophilized particles in PBS and 2. Heprasil, dissolved in PBS and neutralized to pH 7. The hydrogels were incubated at 37°C for 30 minutes to facilitate disulfide bond crosslinking of the open sulfide groups on the cysteine of particles with the open thiol groups in the Heprasil solution. Scaffolds were created in this way for both bone and hyaline cartilage tissue engineering applications. The particle-filled solution can be easily applied to either bone or cartilage defects (using bone or cartilage decellularized tissue, respectively) and creates an environment for osteogenesis or chondrogenesis of the surrounding native pluripotent cells.

The present invention provides a composition of the material components to be utilized in creating a tissue mimic and a combined material/tissue mimic. Furthermore, the present invention provides methods of combining and forming the composition material and methods of using the combination material to mimic tissue in a variety of application cases including, but not limited to, pharmaceuticals, medical devices, 3D printing biomaterial inks, and tissue specific organ cultures.

### References:

A.-V. Do, B. Khorsand, S. M. Geary, A. K. Salem, Advanced healthcare materials 2015, 4, 1742.
   B. N. Brown, S. F. Badylak, Translational Research 2014, 163, 268.
T. Novak, B. Seelbinder, C. M. Twitchell, S. L. Voytik-Harbin, C. P. Neu, Advanced Functional Materials 2016, 26, 5427.
J. E. Barthold, B. M. st. Martin, S. L. Sridhar, F. Vernerey, S. E. Schneider, A. Wacquez, V. L. Ferguson, S. Calve, C. P. Neu, Advanced Functional Materials 2021, 2103355.
   N. A. Sears, D. R. Seshadri, P. S. Dhavalikar, E. Cosgriff-Hernandez, Tissue Engineering Part B: Reviews 2016, 22, 298.
E. A. Aisenbrey, A. Tomaschke, E. Kleinjan, A. Muralidharan, C. Pascual-Garrido, R. R. McLeod, V. L. Ferguson, S. J. Bryant, Macromolecular Bioscience 2018, 18, 1700267.
Y. Lai, H. Cao, X. Wang, S. Chen, M. Zhang, N. Wang, Z. Yao, Y. Dai, X. Xie, P. Zhang, X. Yao, L. Qin, Biomaterials 2018, 153, 1.
K. C. Hung, C. S. Tseng, L. G. Dai, S. hui Hsu, Biomaterials 2016, 83, 156.
   X. Yang, Z. Lu, H. Wu, W. Li, L. Zheng, J. Zhao, Materials Science and Engineering C 2018, 83, 195.
J. S. Choi, H. J. Yang, B. S. Kim, J. D. Kim, J. Y. Kim, B. Yoo, K. Park, H. Y. Lee, Y. W. Cho, Journal of Controlled Release 2009, 139, 2.
J. F. Thompson, C. Bellerjeau, G. Marinick, J. Osio-Norgaard, A. Evans, P. Carry, R. A. Street, C. Petit, G. L. Whiting, ACS Applied Materials and Interfaces 2019, 11, 43337.
   M. Atreya, K. Dikshit, G. Marinick, J. Nielson, C. Bruns, G. L. Whiting, ACS Applied Materials and Interfaces 2020, 12, 23494.
D. Eng, M. Caplan, M. Preul, A. Panitch, Acta Biomaterialia 2010, 6, 2407.

### Definitions:

As used herein, the term "composition" is intended to encompass a product comprising the specified ingredients in the specified amounts, as well as any product which results, directly or indirectly, from combination of the specified ingredients in the specified amounts.

A "safe and effective amount" refers to the quantity of a component that is sufficient to yield a desired therapeutic response without undue adverse side effects (such as toxicity, irritation, or allergic response) commensurate with a reasonable benefit/risk ratio when used in the manner of this invention.

As used throughout the entire application, the terms "a" and "an" are used in the sense that they mean "at least one", "at least a first", "one or more" or "a plurality" of the referenced components or steps, unless the context clearly dictates otherwise. For example, the term "a cell" includes a plurality of cells, including mixtures thereof.

The term "and/or" wherever used herein includes the meaning of "and", "or" and "all or any other combination of the elements connected by said term".
The term "about" or "approximately" as used herein means within 20%, preferably within 10%, and more preferably within 5% of a given value or range.

Other than in the operating examples, or unless otherwise expressly specified, all of the numerical ranges, amounts, values and percentages such as those for amounts of materials, times and temperatures of reaction, ratios of amounts, values for molecular weight (whether number average molecular weight ("Mₙ") or weight average molecular weight ("M_{w}"), and others in the following portion of the specification may be read as if prefaced by the word "about" even though the term "about" may not expressly appear with the value, amount or range. Accordingly, unless indicated to the contrary, the numerical parameters set forth in the following specification and attached claims are approximations that may vary depending upon the desired properties sought to be obtained by the present disclosure. At the very least, and not as an attempt to limit the application of the doctrine of equivalents to the scope of the claims, each numerical parameter should at least be construed in light of the number of reported significant digits and by applying ordinary rounding techniques.

Notwithstanding that the numerical ranges and parameters setting forth the broad scope of the disclosure are approximations, the numerical values set forth in the specific examples are reported as precisely as possible. Any numerical value, however, inherently contain certain errors necessarily resulting from the standard deviation found in their respective testing measurements. Furthermore, when numerical ranges of varying scope are set forth herein, it is contemplated that any combination of these values inclusive of the recited values may be used.

As used herein, the term "comprising" is intended to mean that the products, compositions and methods include the referenced components or steps, but not excluding others. "Consisting essentially of" when used to define products, compositions and methods, shall mean excluding other components or steps of any essential significance. Thus, a composition consisting essentially of the recited components would not exclude trace contaminants and pharmaceutically acceptable carriers. "Consisting of" shall mean excluding more than trace elements of other components or steps.

By suitable for implantation, such as into a patient (human or animal), it is meant that the biocompatible material is sterile/terminally sterilized, endotoxin free, and noncytotoxic. By suitable for use with a 3D printer it is meant that the composition provides a smooth flow on extrusion, can be extruded with a pressure range of 20-100 kPa, and polymerizes after extrusion.

## Claims

1. A composition comprising tissue extracellular matrix particles from biological tissues in combination with thiol-functionalized polymers, wherein the thiol groups on the thiol-functionalized polymers cross-link directly to cysteine residues on the tissue extracellular matrix particles via disulfide bonding thereby forming a biocompatible scaffold.

2. The composition of claim 1, wherein the thiol-functionalized polymers are non-synthetic natural materials selected from the group consisting of collagen, gelatin, hyaluronan, heparin, and combinations thereof.

3. The composition according to claim 1, wherein the thiol-functionalized polymers have a percentage of their functional groups replaced as thiols, wherein the percentage of replacement is selected from the group consisting of 0-15%, 15-30%, 30-60%, and combinations thereof.

4. The composition according to claim 1, wherein the extracellular matrix particles are sourced from natural tissues or organs that are xenogenic, allogenic, or autogenic.

5. The composition according to claim 1, wherein the tissue extracellular matrix particles are sourced from tissue selected from the group consisting of liver, kidney, dermis, cartilage, bone, muscle, ligament, tendon and adipose, and combinations thereof.

6. The composition according to claim 1, wherein the extracellular matrix particles are in the size range selected from the group consisting of less than about 100µm, 100-250µm, and 250-500µm, in the direction of their largest dimension.

7. The composition according to claim 1, wherein the tissue extracellular matrix particles are decellularized, devitalized, lyophilized tissue, or a combination thereof.

8. A method of preparing an organoid or tissue mimic composition comprising the steps of:
providing extracellular matrix particles, which have been morselized via mechanical pulverization of an original tissue source, thereby producing particles that preserve the micro mechanical and biological properties of the original tissue;
combining the extracellular matrix particles with thiol-functionalized polymers in a hydrogel precusor solution; and
incubating the hydrogel precusor solution at about 25°C to about 42°C for about 10 minutes to about 2 hours to facilitate disulfide bonding directly between cysteine residues on the extracellular matrix particles and thiol groups on thiol-functionalized polymers, thereby forming a stable tissue scaffold or 3D structure suitable for tissue repair or 3D organ culture application.

9. The method of preparing an organoid or tissue mimic according to claim 8, wherein the thiol-functionalized polymers have a percentage of their functional groups replaced as thiols, wherein the percentage of replacement is selected from the group consisting of 0-15%, 15-30%, 30-60%, and combinations thereof.

10. The method of preparing an organoid or tissue mimic according to claim 8, wherein the extracellular matrix particles are sourced from tissue selected from the group consisting of liver, kidney, dermis, cartilage, bone, muscle, ligament, tendon and adipose, and combinations thereof, and are in the size range selected from the group consisting of less than about 100µm, 100-250µm, and 250-500µm, in the direction of their largest dimension.

11. The method of preparing an organoid or tissue mimic according to claim 8, wherein the hydrogel precusor solution further comprises one or more external growth factors, region specific anti-inflammatory drugs, or FDA-approved pharmaceutical agents.

## Patentansprüche

1. - Zusammensetzung, umfassend extrazelluläre Gewebematrixpartikel aus biologischen Geweben in Kombination mit Thiol-funktionalisierten Polymeren, wobei die Thiolgruppen an den Thiol-funktionalisierten Polymeren sich über eine Disulfidbindung direkt mit Cysteinresten an den extrazellulären Matrixpartikeln des Gewebes vernetzen und dadurch ein biokompatibles Gerüst bilden.

2. - Zusammensetzung nach Anspruch 1, wobei die Thiol-funktionalisierten Polymere nicht-synthetische natürliche Materialien sind, die ausgewählt sind aus der Gruppe, bestehend aus Kollagen, Gelatine, Hyaluronan, Heparin und Kombinationen davon.

3. - Zusammensetzung nach Anspruch 1, wobei bei den Thiol-funktionalisierten Polymeren ein bestimmter Prozentsatz ihrer funktionellen Gruppen durch Thiole ersetzt ist, wobei der Ersatzprozentsatz ausgewählt ist aus der Gruppe, bestehend aus 0-15 %, 15-30 %, 30-60 % und Kombinationen davon.

4. - Zusammensetzung nach Anspruch 1, wobei die extrazellulären Matrixpartikel aus natürlichen Geweben oder Organen stammen, die xenogen, allogen oder autogen sind.

5. - Zusammensetzung nach Anspruch 1, wobei die extrazellulären Gewebematrixpartikel aus Gewebe stammen, das ausgewählt ist aus der Gruppe, bestehend aus Leber, Niere, Dermis, Knorpel, Knochen, Muskeln, Bändern, Sehnen und Fettgewebe sowie Kombinationen davon.

6. - Zusammensetzung nach Anspruch 1, wobei die extrazellulären Matrixpartikel in dem Größenbereich sind, der ausgewählt ist aus der Gruppe, bestehend aus weniger als etwa 100 µm, 100-250 µm und 250-500 µm in Richtung ihrer größten Abmessung.

7. - Zusammensetzung nach Anspruch 1, wobei die extrazellulären Gewebematrixpartikel dezellularisiertes, devitalisiertes, lyophilisiertes Gewebe oder eine Kombination davon sind.

8. - Verfahren zum Herstellen einer organoiden oder Gewebemimik-Zusammensetzung, umfassend die folgenden Schritte:
Bereitstellung von extrazellulären Matrixpartikeln, die durch mechanische Pulverisierung einer ursprünglichen Gewebequelle zerkleinert wurden, wodurch Partikel erzeugt werden, die die mikromechanischen und biologischen Eigenschaften des ursprünglichen Gewebes bewahren;
Kombinieren der extrazellulären Matrixpartikel mit Thiol-funktionalisierten Polymeren in einer Hydrogel-Vorläuferlösung; und
Inkubieren der Hydrogel-Vorläuferlösung bei etwa 25 °C bis etwa 42 °C über etwa 10 Minuten bis etwa 2 Stunden, um eine Disulfidbindung direkt zwischen Cysteinresten an den extrazellulären Matrixpartikeln und Thiolgruppen an den Thiol-funktionalisierten Polymeren zu ermöglichen, wodurch ein stabiles Gewebegerüst oder 3D-Struktur gebildet wird, das/die sich für Gewebereparatur- oder 3D-Organkultur-Anwendung geeignet ist.

9. - Verfahren zum Herstellen eines Organoids oder einer Gewebemimik nach Anspruch 8, wobei bei den Thiol-funktionalisierten Polymeren ein bestimmter Prozentsatz ihrer funktionellen Gruppen durch Thiole ersetzt ist, wobei der Ersatzprozentsatz ausgewählt ist aus der Gruppe, bestehend aus 0-15 %, 15-30 %, 30-60 % und Kombinationen davon.

10. - Verfahren zum Herstellen eines Organoids oder einer Gewebemimik nach Anspruch 8, wobei die extrazellulären Matrixpartikel aus Gewebe stammen, das ausgewählt ist aus der Gruppe, bestehend aus Leber, Niere, Dermis, Knorpel, Knochen, Muskel, Band, Sehne und Fettgewebe und Kombinationen davon, und in dem Größenbereich sind, der ausgewählt ist aus der Gruppe, bestehend aus weniger als etwa 100 µm, 100-250 µm und 250-500 µm in der Richtung ihrer größten Abmessung.

11. - Verfahren zum Herstellen eines Organoids oder einer Gewebemimik nach Anspruch 8, wobei die Hydrogel-Vorläuferlösung ferner einen oder mehrere externe Wachstumsfaktoren, regionsspezifische entzündungshemmende Arzneimittel oder FDA-zugelassene pharmazeutische Mittel umfasst.

## Revendications

1. - Composition comprenant des microparticules de matrice extracellulaire tissulaire provenant de tissus biologiques en combinaison avec des polymères fonctionnalisés par thiol, dans laquelle les groupes thiol sur les polymères fonctionnalisés par thiol réticulent directement en résidus cystéine sur les particules de matrice extracellulaire tissulaire par liaison disulfure, formant ainsi un échafaudage biocompatible.

2. - Composition selon la revendication 1, dans laquelle les polymères fonctionnalisés par thiol sont des matériaux naturels non synthétiques choisis dans le groupe constitué par le collagène, la gélatine, l'hyaluronane, l'héparine et leurs combinaisons.

3. - Composition selon la revendication 1, dans laquelle les polymères fonctionnalisés par thiol ont un pourcentage de leurs groupes fonctionnels remplacés en tant que thiols, le pourcentage de remplacement étant choisi dans le groupe constitué de 0-15%, 15-30%, 30-60% et leurs combinaisons.

4. - Composition selon la revendication 1, dans laquelle les particules de matrice extracellulaire proviennent de tissus naturels ou d'organes qui sont xénogènes, allogènes ou autogènes.

5. - Composition selon la revendication 1, dans laquelle les particules de matrice extracellulaire tissulaire proviennent de tissus choisis dans le groupe constitué par le foie, le rein, le derme, le cartilage, l'os, le muscle, le ligament, le tendon et le tissu adipeux, et leurs combinaisons.

6. - Composition selon la revendication 1, dans laquelle les particules de matrice extracellulaire sont dans la plage de dimension choisie dans le groupe consistant en moins d'environ 100µm, 100-250µm, et/ou 250-500µm, dans la direction de leur plus grande dimension.

7. - Composition selon la revendication 1, dans laquelle les particules de matrice extracellulaire tissulaire sont des tissus décellularisés, dévitalisés, lyophilisés ou une combinaison de ceux-ci.

8. - Procédé de préparation d'un organoïde ou d'une composition imitant les tissus, comprenant les étapes consistant à :
fournir des particules de matrice extracellulaire qui ont été morcelées par pulvérisation mécanique d'une source de tissulaire d'origine, produisant ainsi des particules qui préservent les propriétés micro-mécaniques et biologiques du tissu d'origine ;
combiner les particules de matrice extracellulaire avec des polymères fonctionnalisés par thiol dans une solution de précurseur d'hydrogel ; et
faire incuber la solution de précurseur d'hydrogel à environ 25°C et à environ 42°C pendant environ 10 minutes à environ 2 heures pour faciliter la liaison disulfure directement entre les résidus cystéine sur les particules de la matrice extracellulaire et les groupes thiol sur les polymères fonctionnalisés par thiol, formant ainsi un échafaudage tissulaire stable ou une structure 3D apte à la réparation tissulaire ou à l'application de culture d'organes en 3D.

9. - Procédé de préparation d'un organoïde ou d'un tissu mimétique selon la revendication 8, dans lequel les polymères fonctionnalisés par thiol ont un pourcentage de leurs groupes fonctionnels remplacés en tant que thiols, le pourcentage de remplacement étant choisi dans le groupe constitué de 0-15%, 15-30%, 30-60% et leurs combinaisons.

10. - Procédé de préparation d'un organoïde ou d'un tissu mimétique selon la revendication 8, dans laquelle les particules de matrice extracellulaire proviennent de tissus choisis dans le groupe constitué par le foie, le rein, le derme, le cartilage, l'os, le muscle, le ligament, le tendon et le tissu adipeux, et leurs combinaisons, et sont dans la plage de dimension choisie dans le groupe constitué par moins d'environ 100µm, 100-250µm, et/ou 250-500µm, dans la direction de leur plus grande dimension.

11. - Procédé de préparation d'un organoïde ou d'un tissu mimétique selon la revendication 8, dans laquelle la solution de précurseur d'hydrogel comprend en outre un ou plusieurs facteurs de croissance externes, des médicaments anti-inflammatoires spécifiques d'une région, ou des agents pharmaceutiques approuvés par la FDA.
